# EUROPEAN PATENT APPLICATION

(11) **EP 3 909 423 A1**
(43) Date of publication of application: **17.11.2021**
(21) Application number: 20181902.6
(22) Date of filing: 24.06.2020
(51) Int. Cl.: A01K 61/40, A01K 63/00, A01K 67/033

(54) **TRANSPORTABLE CONTAINER FOR THE BREEDING OF INVERTEBRATES WITH THREE-DIMENSIONAL PRISMATIC INTERIOR OF GYROID STRUCTURE**

(30) Priority: 12.05.2020 US 202016872399
(71) Applicant: Llecha Galiñanes, Alfredo, 03369 Alicante (ES); Braks, Jorma Gunnar, Waterloo, Ontario N2L 5M9 (CA)
(72) Inventor: Llecha Galiñanes, Alfredo, 03369 Alicante (ES); Braks, Jorma Gunnar, Waterloo, Ontario N2L 5M9 (CA)
(74) Representative: Lahidalga de Careaga, Jose Luis

(57) **Abstract**

Transportable container for the breeding of invertebrates with a three-dimensional prismatic interior of gyroid structure of continuous development characterized by being made up of a container of straight rectangular parallelepiped shape (1) which has a top cover (2) in its upper part and two rings in the middle of its side faces (3), and where in the lower third of the container's (1) height there is a perimetral elastic element (4) to be able to help the assembly vibrate without causing breakage; bellow the perimetral elastic element (4) and in the lateral faces there are two couplings (5) to fix a hose; at the outer side of the container (6) there is a sliding profile (7), which is located on the inside of the container (1) and is positioned on the inner side of the container; there is a waste collection tray (8) that runs along the surface of the profile, which has two semi-circular tabs (9) to allow the removal of the tray; at the bottom of the container (1) there are four legs (10) made up of two L-shaped plates that start from the four corners of the container (1) and rest on a transport platform (11) carried by a mobile vehicle; and there is a central element that occupies 70% of the container formed The central element that occupies 70% of the container is a three-dimensional prismatic interior of gyroid structure of continuous development (12), built by a 3D printer in plastic material, where the internal or empty part of this body is over 98% of it.

## Description

### OBJECT OF THE INVENTION

The invention presented refers to a transportable container for the breeding of invertebrates with a three-dimensional prismatic interior of gyroid structure of continuous development, so that one can always move from two spots of said structure without leaving its surface, and which presents an important series of improvements over existing technology.
We define as invertebrates the larvae, such as the black soldier fly (BSF) or Hermetia Illucens, larvae of T. molitor and adults, crickets, and several species of terrestrial and aquatic annelids.

Fish-based food and soy-based food are the main sources of protein for the production of animal food, however both sources compete with the production of food for human consumption. In addition, the availability of fish-based food is becoming more limited due to the over-exploitation of marine resources, leading to a constant increase in the price of both protein sources. As a result, the search for sustainable protein sources has focused on the use of invertebrate based food, mainly insects, because of its high nutritional content as an ingredient in the formulation of animal foods and to a lesser extent, insects themselves are also prepared for human consumption.

As a result of new laws and regulations for the use of insects as edible food and a substitute for fish-based food and soy-based food, insect factories and production groups have emerged worldwide in recent years. One species of insect in particular, called the black soldier fly or BSF, has attracted attention because it has great potential, mainly because its larvae feed on many organic sources and organic residues of plant and animal origin. The ability of the black soldier fly to rapidly transform these organic residues into high quality nutrients has opened up commercial opportunities for the production of high protein food which is cheaper and an alternative to fish-based food and soy-based food. According to a study published by Meticulous Research ®, the market for products derived from the black soldier fly (BSF) production will reach a value of $2.57 billion by 2030.

The existing industrial production systems for the black soldier fly are basically based on the cultivation of larvae in flat containers. But all the known containers used in this industry have a number of problems:
Conventional BSF breeding is done on flat trays or similar containers, always compromised by the height of the culture measured from the surface of the culture to the bottom of the rearing container. Generally, the maximum depth is considered in the range of 10-15 cm.
Above this height, population pressure, crushing, asphyxiation, and excessive heat generation by the larvae and microorganisms living in the culture itself, causes high mortality of the larvae.
To try to overcome these problems, several technical solutions were tried but without solving the main problem which is the depth of the containers.

The object of the invention is a solution which will significantly reduce the disadvantages and inconveniences of the above technique by minimizing the mixing of food and excreta to the invertebrates and providing a natural habitat where the insects can hide and feed at any depth.

This solution allows for the breading of invertebrates with a greater volume at a lower cost due to the much smaller floor area of a plant, the ease of automation and the fact that the larvae are easily separated from the food remains.

### FIELD OF THE INVENTION.

The invention falls within the auxiliary industry of breading larvae and invertebrates and very essentially in the manufacture of containers and accessories for breading them.

### BACKGROUND OF THE INVENTION

As we have previously indicated, conventional breading is carried out in flat trays or similar containers, always compromised by the height of the culture measured from the surface of the culture to the bottom of the breeding container. Generally, the maximum depth is considered in the range of 10-15 cm. Above this height, mortality increases and affects the entire colony.
To try to overcome the problems several technical solutions were tried:
1. Adding various types of inert substrate mixed with food. The success was not sufficient to increase the maximum depth of the crop,
2. Increase the density of larvae and incorporate food on alternate days. Apparently, it had little success
3. Increase the aeration of the culture. Also, with little success.
4. Testing different forms of breeding containers. Including cylinders or tubes placed horizontally, triangular shapes, etc. But the pressure of the crop does not depend on the shape of the container, but on the total mass of the larvae and their food and the depth of the culture.
5. Rotate the culture with a constant or variable rotation speed. For example, using an auger conveyor. This solution is not operational
6. Reduction of population or culture pressure by using low height containers. This is the universal solution adopted on an industrial scale, but a BSF breeding plant using 45,000 trays costs $16 million and where basically, each tray is filled with 3-day-old larvae. Transported by conveyor belts and lifted and stacked in "towers", whose height is limited to the weight of the entire column, and can cause the "towers" to collapse on their own; or if a low-magnitude earthquake occurs in the area where the plant was built.

The object of the invention is a solution which significantly reduces the disadvantages and inconveniences of the above technique and the key element is its ability to minimize the mixing of food and excreta to the invertebrates and provide a natural habitat where they can hide and feed at any depth and reduce population pressure.

The container described presents a series of improvements that solve all the previous problems due to the following technical characteristics and mainly due to both its external structure and the internal structure formed by a three-dimensional prismatic body of continuous development similar a defined gyroid body.

The improvements obtained are:
The internal structure is former by a three-dimensional prismatic interior of gyroid structure of continuous development The internal structure has an unlimited surface area, the invertebrates can move from one spot (x1, y1, z1) of the structure to any other spot (x2, y2, z2,) without leaving its surface.
- The internal structure has a larger surface area by volume than other structures.
- The internal structure allows maintaining a higher density of invertebrates by volume than other structures.
- The inner structure allows invertebrates to feel protected from predators.
- The internal structure allows invertebrates to be protected from direct light.
- The internal structure allows invertebrates to rest safely in the absence of food and to remain in diapause.
- The internal structure prevents the invertebrates from losing water, and even from dehydrating, by keeping the moisture inside more efficiently than other structures.
- The internal structure allows invertebrates to feed on fresh food not contaminated by their own feces and leachate. Feces and leachate rarely mix with food and are deposited directly on the bottom of the container.
- The three-dimensional structure of continuous development can withstand the great pressure generated by the mass of invertebrates and their food and the force of gravity.
- And all of this means that unlike other structures, the three-dimensional of continuous development structure allows invertebrates to move and feed at any height in the structure without being exposed to the great pressure generated by the mass of individuals, food and the force of gravity.
- The interior structure also allows the entrance of atmospheric or forced air allowing the invertebrates to breathe at any level of the structure.
- The interior structure allows the invertebrates to stay inside preventing escapes.

And at industrial level the structure with the invertebrates inside, can be harvested by gravity lifting the structure and waiting for the invertebrates to fall to the bottom of the container, vibration can be used to accelerate the process.

Also the three-dimensional prismatic of continuous development structure can be moved with the invertebrates inside to devices where they are harvested using centrifugal force, vibration or gravity.
In addition, the structure can be washed and sanitized with hot water, hot air, or steam and reused multiple times unlike other structures.
Also, the container can be installed on trucks, saving food transportation costs, while the mass of invertebrates grows during transportation, and saving energy costs by using the engine as a source of heat and cold.
The inventor does not know of any transportable container for the breeding of invertebrates that has the technical, constructive characteristics of efficiency and savings that this invention presents.

### BRIEF DESCRIPTION OF THE INVENTION

The transportable container for the breeding of invertebrates with an with a three-dimensional prismatic interior of gyroid structure of continuous development is made up of a straight rectangular parallelepiped-shaped container opened by the bases that has a top cover on its upper part and on the upper middle part of the side faces a ring that will serve for the lifting and handling of the container.

In the lower third of the container's height there is an elastic element around its perimeter which will serve to make the whole container vibrate without causing breakage and which will serve to accelerate the harvesting by gravity and vibration, so that the invertebrates fall by gravity.

Bellow the perimetral elastic element, in the lateral faces of the container there are two couplings to fix an external hose. These hoses serve to introduce heat or cold, possibly from the vehicle transporting the containers and accelerate the maturation processes.
In the lower part of the container, there are sliding profiles located on the inside of the container and placed on the inner side faces of the container, on the surface of which the waste collection tray runs, its very definition identifies its function. The tray has two semi-circular tabs to allow the tray to be removed.

At the bottom of the container, there are four legs made up of L-shaped plates that are placed at the four corners of the container and rest on the transport platform possibly carried by a mobile vehicle.
The central element, which occupies 70% of the container, is a three-dimensional prismatic interior of gyroid structure of continuous development built by a 3D printer in plastic material. A variety of invertebrates can be grown in an artificial environment, while the amount of food is controlled to allow the invertebrates to feed as needed while providing a place for the invertebrates to move freely and cleanly from the food. The key element is this three-dimensional prismatic body as the feature of its design and its ability to minimize the mixing of food and feces and the invertebrates and to provide a natural habitat where they can hide at any depth under atmospheric pressure.
This invention refers to a container for the breeding of invertebrates with a novel matrix or chamber that utilizes fundamental principles of food supply while keeping food and feces separate from growing larvae. The design of the matrix provides means to allow the larvae to move freely without being suffocated, crushed or overheated while consuming the food.

### DESCRIPTION OF THE DRAWINGS.

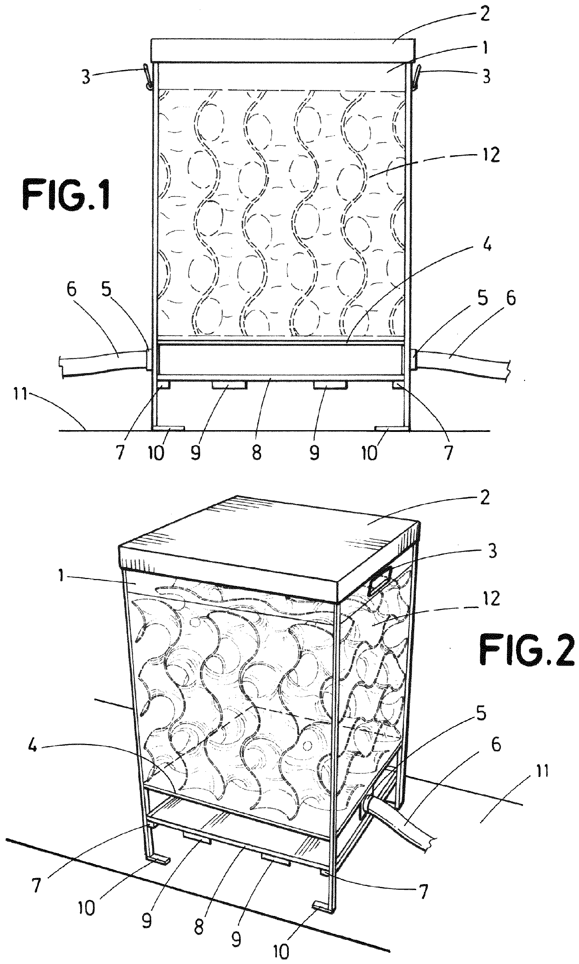

In order to enhance the description and helping to a better understanding of the characteristics of the invention, this description is accompanied, as an integral part of it, by a sheet of drawings, in which identical elements are indicated with identical references and where, for illustrative purposes and without limitation, the following has been represented
FIGURE N° 1. Side view in diagram of the transportable container.
FIGURE N° 2. Isometric view of the transportable container

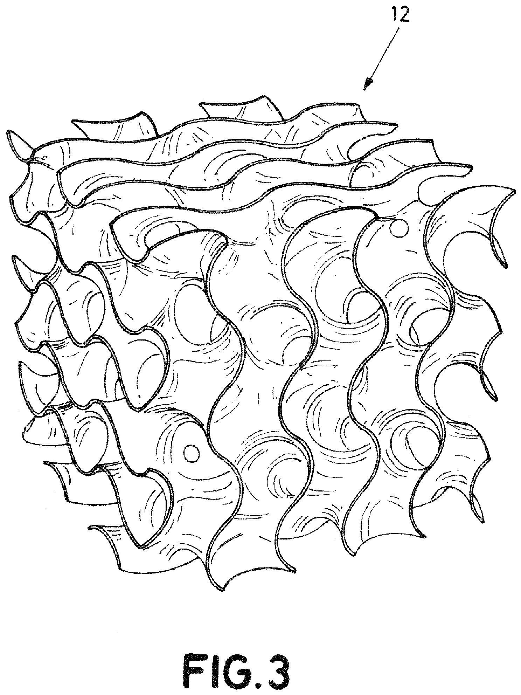

FIGURE N° 3. The three-dimensional prismatic interior of gyroid structure of continuous development.

And in these figures the following elements are identified
(1).- container,
(2).- top cover of the container,
(3).- container lifting rings,
(4).- perimeter elastic element,
(5).- Coupling for fixing the external hose,
(6).- outer hose,
(7) .- sliding profile of the waste collection tray,
(8).- waste collection tray,
(9).- waste tray recovery tabs,
(10).- container legs,
(11).- transport platform,
(12) . three-dimensional prismatic interior of gyroid structure of continuous development.

### PREFERENTIAL REALIZATION OF THE INVENTION

The transportable container for breeding invertebrates with a three-dimensional prismatic interior of gyroid structure of continuous development is made up of a rectangular straight parallelepiped-shaped container (1) with a top cover (2) in the upper part and rings (3) in the middle of the side faces, which will serve for lifting and handling the container (1).

In the lower third of the container's (1) height there is a perimeter elastic element (4) which will be of use when the assembly vibrates preventing breakage and which will serve to accelerate the harvest by gravity and vibration, so that the invertebrates fall by gravity.

Bellow of the perimeter elastic element, there are two couplings (5) on the side faces to fix an external hose (6). These hoses serve to introduce heat or cold from the vehicle transporting the containers.

At the lower part of the container, there are two sliding profiles (7) located on the inside of the container (1) and placed on the interior side faces of the container, on the surface of which the waste collection tray (8) runs, the very definition of which identifies its function. This tray (8) has two semi-circular tabs (9) to enable the tray to be removed.

At the bottom of the container (1) there are four legs (10) made up of L-shaped plates which start from the four corners of the container (1) and rest on the transport platform (11) carried by a mobile vehicle.

The central element that occupies 70% of the container is a three-dimensional prismatic interior of gyroid structure of continuous development (12), built by a 3D printer in plastic material.
- The gyroid surface can be trigonometrically approximated by the equation: sin x cos y + sin y cos z + sin z cos x = 0
-The thickness of the surface of the gyroid structure may be between 0.5 mm and 1.5 mm.
. The gyroid structure separates space into two oppositely congruent labyrinths of passages where passages emerge at 70.5 degree angles to any given channel as it is traversed, The gyroid structure contains neither straight lines nor planar symmetries.
The internal or empty part of this medium or matrix is over 98%, while providing horizontal and vertical pathways for the larvae to travel without fear of being crushed by the high pressure created by the live larvae above.
Adult invertebrates reared within the three-dimensional prismatic body (12) so that one can always move from P1 (x1,y1,z1) to P2 (x2,y2,z2) without leaving the surface, can also be fed fresh, uncrushed vegetables and fruit, and females are easily attracted to the "egg traps" located on top of the material; the use of this material will then prevent females from laying eggs in the substrate.
In addition, since the material used in the manufacture of the three-dimensional prismatic interior structure of continuous development body is plastic (12), the body is reusable and can be washed and sanitized.
It should be made clear, with sufficient description of the nature of the invention and the manner in which it is put into practice, that the provisions indicated above and represented in the drawings attached are susceptible of modification of detail in so far as they do not alter the fundamental principles set forth in the preceding paragraphs and summarized in the following claims.

## Claims

1. Transportable container for the breeding of invertebrates with a three-dimensional prismatic interior of gyroid structure of continuous development **characterized by** being made up of a container of straight rectangular parallelepiped shape (1) which has a top cover (2) in its upper part and two rings in the middle of its side faces (3), and where in the lower third of the container's (1) height there is a perimetral elastic element (4) to be able to help the assembly vibrate without causing breakage; bellow the perimetral elastic element (4) and in the lateral faces there are two couplings (5) to fix a hose; at the outer side of the container (6) there is a sliding profile (7), which is located on the inside of the container (1) and is positioned on the inner side of the container; there is a waste collection tray (8) that runs along the surface of the profile, which has two semi-circular tabs (9) to allow the removal of the tray; at the bottom of the container (1) there are four legs (10) made up of two L-shaped plates that start from the four corners of the container (1) and rest on a transport platform (11) carried by a mobile vehicle; and there is a central element that occupies 70% of the container formed by a three-dimensional prismatic interior of gyroid structure of continuous development (12), built by a 3D printer in plastic material, where the internal or empty part of this body is over 98% of it.

2. Transportable container for the breeding of invertebrates with a three-dimensional prismatic interior of gyroid structure of continuous development accordingly to claim1 and characterized because:
the gyroid surface can be trigonometrically approximated by the equation: sin x cos y + sin y cos z + sin z cos x = 0;
the thickness of the surface of the gyroid structure may be between 0.5 mm and 1.5 mm.
the gyroid structure separates space into two oppositely congruent labyrinths of passages, where passages emerge at 70.5 degree angles to any given channel as it is traversed;
the gyroid structure contains neither straight lines nor planar symmetries.
